# EUROPEAN PATENT APPLICATION

(11) **EP 0 564 193 A1**
(43) Date of publication of application: **06.10.1993**
(21) Application number: 93302389.7
(22) Date of filing: 26.03.1993
(51) Int. Cl.: C07D 221/10, C07D 215/22, C07D 457/00, C07D 221/16, C07B 35/00

(54) **Heteroannulation process**

(30) Priority: 03.04.1992 US 863574
(71) Applicant: ELI LILLY AND COMPANY, Indianapolis, Indiana 46285 (US)
(72) Inventor: Audia, James Edmund, Indianapolis, Indiana 46278 (US); Lawhorn, David Ernest, Greenfield, Indiana 46140 (US)
(74) Representative: Tapping, Kenneth George

(57) **Abstract**

A heteroannulation process for preparing piperidones and tetrahydropyridones by reacting an appropriately substituted enamine with an acryloyl derivative wherein a leaving group occupies the carbonyl; a process for cleaving a 1-phenylethyl group from an acylated amino group or an heterocyclic nitrogen atom adjacent to a carbonyl group, particularly that on the nitrogen atom of the products of the heteroannulation process, is also provided.

## Description

The present invention belongs to the field of synthetic organic chemistry and pharmaceutical chemistry, and provides a new process for the formation of piperidones and tetrahydropyridones.

Pharmaceutical chemists have found a great number of interesting and valuable compounds among the nitrogen-containing heterocycles, and among compounds made up of multiple heterocyclic rings, some of which contain nitrogen. The 6-membered piperidone and tetrahydropyridone rings, and the corresponding piperidines and tetrahydropyridines which can be obtained by their reduction by standard methods, are frequently found in such heterocyclic compounds, either alone or in spiro or fused constructions. It is also frequently found that the optimum stereochemistry of asymmetric compounds is important in obtaining optimum activity from pharmaceutical compounds. The present invention provides a convenient 2-step process for forming such piperidone and tetrahydropyridone compounds, which are also useful intermediates for the formation of piperidines and tetrahydropyridines.

This invention provides a heteroannulation process for preparing a non-racemic compound of the formula 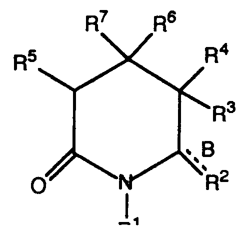
wherein
R¹ represents a chiral auxiliary; line B completes a double bond;
R² represents C₁-C₆(alkylidene); C₁-C₆ aryl(alkylidene); C₃-C₈ cyclo(alkylidene); C₄-C₁₂ cycloalkyl(alkylidene); wherein aryl, alkylidene, and cyclo(alkyl or alkylidene) groups may be substituted with C₁-C₃ alkyl; halo; amino; C₁-C₃ haloalkyl; C₁-C₃ alkyl- or dialkylamino; hydroxy; C₁-C₃ alkoxy; C₁-C₃ alkylthio; cyano; alkoxycarbonyl; oxo; aryloxycarbonyl; alkylsulfonyl; alkylsulfinyl; N- or N,N-dialkylaminocarbonyl; arylaminocarbonyl; alkanoyl or aroyl;
R³ represents C₁-C₆ alkylthio; C₁-C₆ alkoxy; C₁-C₆ alkyl; aryl; C₁-C₆ arylalkyl; C₃-C₈ cycloalkyl; or C₄-C₁₂ cycloalkylalkyl; alkanoyl; aroyl; alkoxycarbonyl; or aryloxycarbonyl; wherein aryl, alkyl and cycloalkyl groups may be substituted with C₁-C₃ alkyl; halo; amino; C₁-C₃ haloalkyl; C₁-C₃ alkyl- or dialkylamino; hydroxy; C₁-C₃ alkoxy; C₁-C₃ alkylthio; cyano; alkoxycarbonyl; oxo; aryloxycarbonyl; alkylsulfonyl; alkylsulfinyl; N- or N,N-dialkylaminocarbonyl; arylaminocarbonyl; alkanoyl or aroyl;
R² and R³ may combine to complete a two-, three- or four-fused-ring structure containing 0-6 heteroatoms chosen from N, O and S; which may be substituted with C₁-C₃ alkyl; halo; amino; C₁-C₃ haloalkyl; C₁-C₃ alkyl- or dialkylamino; hydroxy; C₁-C₃ alkoxy; C₁-C₃ alkylthio; cyano; alkoxycarbonyl; oxo; aryloxycarbonyl; alkylsulfonyl; alkylsulfinyl; N- or N,N-dialkylaminocarbonyl; arylaminocarbonyl; alkanoyl or aroyl;
R⁴ represents C₁-C₆ alkylthio; C₁-C₆ alkoxy; C₁-C₆ alkyl; aryl; C₁-C₆ arylalkyl; C₃-C₈ cycloalkyl; or C₄-C₁₂ cycloalkylalkyl; wherein aryl, alkyl and cycloalkyl groups may be substituted with C₁-C₃ alkyl; halo; amino; C₁-C₃ haloalkyl; C₁-C₃ alkyl- or dialkylamino; hydroxy; C₁-C₃ alkoxy; C₁-C₃ alkylthio; cyano; alkoxycarbonyl; oxo; aryloxycarbonyl; alkylsulfonyl; alkylsulfinyl; N- or N,N-dialkylaminocarbonyl; arylaminocarbonyl; alkanoyl or aroyl;
R³ and R⁴ combine to form a two-, three- or four-ring structure containing 0-6 heteroatoms chosen from N, O and S; which may be substituted with C₁-C₃ alkyl; halo; amino; C₁-C₃ haloalkyl; C₁-C₃ alkyl- or dialkylamino; hydroxy; C₁-C₃ alkoxy; C₁-C₃ alkylthio; cyano; alkoxycarbonyl; oxo; aryloxycarbonyl; alkylsulfonyl; alkylsulfinyl; N- or N,N-dialkylaminocarbonyl ; arylaminocarbonyl; alkanoyl or aroyl; provided that when R³ combines with R⁴, R² does not combine with R³;
R⁵ represents hydrogen; C₁-C₆ alkylthio; C₁-C₆ alkoxy; C₁-C₆ alkyl; aryl; C₁-C₆ arylalkyl; C₃-C₈ cycloalkyl; or C₄-C₁₂ cycloalkylalkyl; wherein aryl, alkyl and cycloalkyl groups may be substituted with C₁-C₃ alkyl; halo; amino; C₁-C₃ haloalkyl; C₁-C₃ alkyl- or dialkylamino; hydroxy; C₁-C₃ alkoxy; C₁-C₃ alkylthio; cyano; alkoxycarbonyl; oxo; aryloxycarbonyl; alkylsulfonyl; alkylsulfinyl; N- or N,N-dialkylaminocarbonyl; arylaminocarbonyl; alkanoyl or aroyl;
R⁶ represents hydrogen; C₁-C₆ alkylthio; C₁-C₆ alkoxy; C₁-C₆ alkyl; aryl; C₁-C₆ arylalkyl; C₃-C₈ cycloalkyl; or C₄-C₁₂ cycloalkylalkyl; wherein aryl, alkyl and cycloalkyl groups may be substituted with C₁-C₃ alkyl; halo; amino; C₁-C₃ haloalkyl; C₁-C₃ alkyl- or dialkylamino; hydroxy; C₁-C₃ alkoxy; C₁-C₃ alkylthio; cyano; alkoxycarbonyl; oxo; aryloxycarbonyl; alkylsulfonyl; alkylsulfinyl; N- or N,N-dialkylaminocarbonyl; arylaminocarbonyl; alkanoyl or aroyl;
R⁷ represents hydrogen; C₁-C₆ alkylthio; C₁-C₆ alkoxy; C₁-C₆ alkyl; aryl; C₁-C₆ arylalkyl; C₃-C₈ cycloalkyl; or C₄-C₁₂ cycloalkylalkyl; wherein aryl, alkyl and cycloalkyl groups may be substituted with C₁-C₃ alkyl; halo; amino; C₁-C₃ haloalkyl; C₁-C₃ alkyl- or dialkylamino; hydroxy; C₁-C₃ alkoxy; C₁-C₃ alkylthio; cyano; alkoxycarbonyl; oxo; aryloxycarbonyl; alkylsulfonyl; alkylsulfinyl; N- or N,N-dialkylaminocarbonyl; arylaminocarbonyl; alkanoyl or aroyl; or
R⁶ and R⁷ combine to form a two-, three- or four-ring structure containing 0-6 heteroatoms chosen from N, O and S; which may be substituted with C₁-C₃ alkyl; halo; amino; C₁-C₃ haloalkyl; C₁-C₃ alkyl- or dialkylamino; hydroxy; C₁-C₃ alkoxy; C₁-C₃ alkylthio; cyano; alkoxycarbonyl; oxo; aryloxycarbonyl; alkylsulfonyl; alkylsulfinyl; N- or N,N-dialkylaminocarbonyl; arylaminocarbonyl; alkanoyl or aroyl;
comprising reacting an enamine of the formula 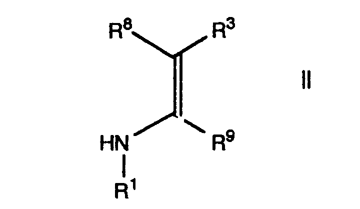
wherein R⁸ represents R^{4,} R⁹ represents R², provided that when R² represents an alkylidene-linked group, R⁹ represents the corresponding alkyl-linked group;
with an acryloyl derivative of the formula 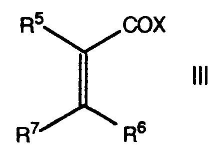
wherein X is a leaving group.

The invention also provides a process for cleaving a 1-phenylethyl group from an acylated amino group or from a heterocyclic nitrogen atom adjacent to a carbonyl group comprising reacting the 1-phenylethyl-substituted compound with trifluoroacetic acid.

In this document, all temperatures are expressed in degrees Celsius and all expressions of percentage, concentration, proportion and the like are in weight units unless otherwise specified.

In the above general formulae, the general chemical terms are used in their usual meanings and context. Some specific exemplification will be mentioned, to assure clarity.

The term "C₁-C₆ alkyl" refers to groups such as methyl, propyl, hexyl, 4-methylpentyl and the like, which, it will be understood, may be branched in any reasonable manner. The term "C₁-C₆ phenylalkyl" includes such groups having a phenyl group attached at a terminal or any other carbon atom. The term "C₃-C₈ cycloalkyl" includes groups such as cyclopropyl, cyclopentyl and cyclooctyl, and the term "C₄-C₁₂ cycloalkylalkyl" includes groups having such cycloalkyls attached to an alkyl group of such size as to give a total number of carbon atom up to twelve.

The term "C₁-C₃ alkyl" includes methyl, ethyl, propyl and isopropyl, and "C₁-C₃ haloalkyl" includes such groups substituted with iodine, bromine, chlorine or fluorine atoms. Those halogen atoms are included in the term "halo". Alkylamino and dialkylamino groups may be formed by free substitution with C₁-C₃ alkyl groups, and C₁-C₃ alkoxy and alkylthio include such alkyl groups linked through an oxygen or sulfur atom, respectively.

The term "aryl" includes, particularly, phenyl, but also includes such carbocyclic aryl groups as napthyl and the like, and includes such simple heterocyclic aryl groups as pyridine, pyrazine, quinoline, and the like. The term "alkanoyl" includes alkyl groups linked through a carbonyl, and the term "aroyl" includes aryl groups linked through a carbonyl group. Alkyl- and dialkylaminocarbonyl groups and arylaminocarbonyl groups include typical carboxamido groups wherein the nitrogen is substituted with alkyl or aryl, respectively.

Alkoxycarbonyl and aryloxycarbonyl groups are made up of alkoxy or aryloxy groups linked through a carbonyl, respectively.

Where the maximum size of alkyl groups which are part of constructions such as alkoxycarbonyl, and the like, is not specified, it will be understood that such groups may comprise as many as six carbon atom, or even more so long as the function of the present process is not impeded by said groups.

Aryl, alkyl, alkylidene, and cycloalkyl groups, where so stated in the general formula, may be substituted with from one named substituent group up to full substitution of the ring group, and combining the different types of substituent groups if desired, bearing in mind the limitations imposed by steric hindrance and the like which are understood by organic chemists.

It will be observed that the products of the heteroannulation process have a double bond exo to the ring, line B. Accordingly, the group R² is linked by a double bond, forming an alkylidene group as the skilled reader will understand.

The groups R² and R³ may combine to complete a second ring, fused to the piperidone ring, which may further contain one or more N, O or S atoms. As described in the formula, that ring may be part of a larger construction making up as many as four, or even more, fused rings in all and forming, for example, an ergoline, benzoquinone, azepino-tetrahydropyridone, and related multiple-ring molecules. It will be understood that the importance and the utility of the present invention is provided by its facile synthesis of the piperidone ring, and the remainder of the molecule to be constructed is not particularly material to the application of the present invention.

Further, the groups R³ and R⁴ may combine to form a ring which is attached spiro to the piperidone or tetrahydropyridone ring, and which may be further attached to additional rings to form a linked structure which may contain as many as four, or more, rings and contain as many as six heteroatoms chosen from N, O or S. Further, the groups R⁶ and R⁷ may also combine to form a spiro-linked ring which may be further combined with additional rings. It will be further understood that the various linked- or fused-ring products of the present process may be substituted with additional substituents where such substitution is not prevented by unsaturation or steric hindrance.

While the full scope of the heteroannulation products described above may be advantageously prepared by the present process, certain of the products are particularly desirably prepared. The following paragraphs and subparagraphs describe particularly advantageous products of the heteroannulation.
R¹ represents phenylalkyl;
R¹ represents C₁-C₃ phenylalkyl;
R¹ represents phenylalkyl or cycloalkyl substituted with alkyl, halo, hydroxy or alkoxy;
R² and R³ combine to form a fused-ring structure;
R² and R³ combine to form a carboxyclic fused-ring structure;
R² and R³ combine to complete a fused-ring-structure containing 1-3 heteroatoms;
R² and R³ combine to complete a fused-ring-structure containing 1-3 heteroatoms, which may be substituted with alkyl, halo, alkoxycarbonyl, hydroxy or alkoxy;
R² represents alkylidene, phenylalkylidene, cycloalkylidene or cycloalkylalkylidene, wherein phenyl and cycloalkyl groups may be substituted with alkyl, halo, haloalkyl or alkoxy;
R³ represents C₁-C₃ alkyl, phenyl, C₁-C₃ phenylalkyl, cycloalkyl or cycloalkylalkyl, wherein phenyl and cycloalkyl groups may be substituted with alkyl, halo, haloalkyl, or alkoxy;
R⁴ represents alkyl, phenyl, C₁-C₃ phenylalkyl, cycloalkyl, cycloalkylalkyl or alkoxycarbonyl, wherein phenyl and cycloalkyl groups may be substituted with alkyl, halo, haloalkyl or alkoxy groups;
R³ and R⁴ combine to form a 2-, 3- or 4-ring structure;
R⁵ represents hydrogen, hydroxy, alkyl, C₁-C₃ phenylalkyl, cycloalkyl or cycloalkylalkyl, wherein phenyl and cycloalkyl groups may be substituted with alkyl, halo, haloalkyl or alkoxy groups;
R⁶ represents hydrogen, hydroxy, alkyl, C₁-C₃ phenylalkyl, cycloalkyl or cycloalkylalkyl, wherein phenyl and cycloalkyl groups may be substituted with alkyl, halo, haloalkyl or alkoxy groups;
R⁷ represents hydrogen, hydroxy, alkyl, C₁-C₃ phenylalkyl, cycloalkyl or cycloalkylalkyl, wherein phenyl and cycloalkyl groups may be substituted with alkyl, halo, haloalkyl or alkoxy groups;
R⁶ and R⁷ combine to form a 2-, 3- or 4-ring structure.

The cleavage process of the present invention is advantageously applied to the removal of the 1-phenylethyl group from amino groups which are pendant from organic molecules of all types. For example, arylamines such as those in which the amino group is linked to phenyl, napthyl, benzimidazole, anthracene, quinoxaline, pyridazine, piperidine, fused piperidine and like aryl structures is advantageous and preferred in the context of the present cleavage process. It will be understood, however, that the amino nitrogen must be acyl-substituted when the cleavage process is carried out. It will also be observed that the cleavage process may be applied to compounds wherein the 1-phenylethyl group is linked to a nitrogen atom which is part of a heterocyclic molecule, so long as a carbonyl group is adjacent to the nitrogen. The examples below show an advantageous use of the cleavage in the preparation of unsaturated benzoquinolines and ergot-related molecules. The reader will understand the cleavage is equally advantageously applied to N-1-phenylethyl derivatives of any of a great variety of heterocyclic compounds, including piperidine, diazaspiroundecane, pyranopyridine, thiopyranopyridine, benzodiazepine, benzoxazepine, benzothiazepine, diazaspirotridecane, benzoxazine, pyridooxazine, benzoxadiazine, benzothiadiazine, cycloheptatriazole and the like, wherein the necessary carbonyl is adjacent the nitrogen.

The identity of the enamine and acryloyl derivative predict the identity of the product. The R¹, R³, R⁵, R⁶ and R⁷ substituents of the starting compounds carry through into the product as the skilled chemist would predict. R⁸ corresponds to R⁴. Similarly, R⁹ corresponds to R², except that when R² in the final product is to be an alkylidene-linked group, then R⁹ in the starting compound will represent the corresponding alkyl-linked group. For example, if R² is to be cyclohexylmethylidene, R⁹ is cyclohexylmethyl.

When linked-ring structures are to be present in the product, the corresponding structure is present in the starting compound. For example, if R² and R³ are to form a ring, R³ and R⁹ in the starting enamine correspondingly form a ring. If R³ and R⁴ are to form a spiro-linked ring in the product, R³ and R⁸ in the enamine form the same ring; and if R⁶ and R⁷ are to form a spiro-linked ring in the product, R⁶ and R⁷ in the acryloyl derivative will do the same.

The group X is a leaving group such as are commonly used in organic chemistry. Preferred leaving groups are chlorine and bromine atoms. Additional leaving groups which may be used include sulfonates such as toluenesulfonate and methane sulfonate.

The process conditions for carrying out the heteroannulation are extremely mild. In most instances, it will be found that excellent yields are obtained in short periods of time at temperatures in the range of ambient. For example, temperatures from about 0° to about 150° are used, and reaction times in the range of from about a few minutes to, at maximum, a few hours, are sufficient. The reaction medium is preferably a biphasic mixture of a convenient organic solvent and an aqueous solution of a mild base. Useful solvents include, for example, haloalkanes, ethers including tetrahydrofuran, and nitriles including acetonitrile. Preferred mild bases are alkali metal carbonates and bicarbonates; more highly basic reagents such as alkali and alkaline earth metal hydroxides and the like may be used in some cases, but the bicarbonates are typically preferred. The process may also be performed without a base if desired.

The products of the heteroannulations are readily isolated by conventional process steps. As is discussed elsewhere, the use of a heteroannulation process in which R¹ is a single-isomer 1-phenylethyl (α-methylbenzyl) group, which is cleaved after the heteroannulation by the presently disclosed cleavage process, provides a particularly clean synthesis of single-isomer forms of the heteroannulation product.

It will be understood that the products of the present heteroannulation process may be used as such to take advantage of their biological activity, or they may be used as intermediates in additional processes to prepare active compounds. For example, the compounds may be reduced, further oxidized, halogenated and alkylated; additional ring structures may be constructed around them or attached to them, and they may be linked to entirely different structures, such as amino acid constructions.

The second aspect of the present invention, the cleavage process, is of extremely broad application. It will be understood that the 1-phenylethyl group is frequently used as a chiral auxiliary in the synthesis of numerous types of asymmetric or optically active compounds. Use of the chiral auxiliary makes it convenient to separate optical isomers. Conventionally, the chiral auxiliary is removed after the step in which it performs its designated purpose.

The present cleavage is a particularly clean way to remove the 1-phenylethyl group from amines or from a nitrogen atom contained in a heterocyclic ring. It has been observed that the present cleavage will remove that auxiliary, while maintaining the chiral identity of the intermediate. When the process is used to cleave the 1-phenylethyl group from an amino group, it is necessary that the amino group be acylated. Often the advantageous acyl group is one which is easily removed after the cleavage is performed. The acyl group used for this purpose may advantageously be any of the commonly used amino-protecting groups, as taught by Greene, "Protective Groups in Organic Synthesis", Wiley and Sons, New York, New York, 1981, Chap. 7. For example, the benzyloxycarbonyl group, the 4-methoxybenzoyloxy-carbonyl group, the 4-nitrobenzyloxycarbonyl group, the 2,2,2-trichloroethoxycarbonyl group and the like are commonly used and are useful here.

The cleavage process is carried out by the simple reaction of the 1-phenylethyl-substituted intermediate with trifluoroacetic acid, at a moderate temperature. For example, temperatures in the range of from ambient temperature to 100° may be used; it is preferred to carry out the reaction at from ambient temperature to about 60°. Ordinarily, the reaction will be found to be complete in a period of time from several minutes to a few hours.

An unexpected advantage of the present cleavage process is its freedom from unwanted side reactions. For example, some prior art methods of cleaving such compounds with strong acids have the property of reducing an available double bond in the intermediate. Such a side reaction does not occur during cleavage of the present invention. Accordingly, a preferred application of the present cleavage is to a compound wherein a double bond is located close to the 1-phenylethyl-substituted nitrogen, particularly when the double bond is alpha, beta to the nitrogen atom. Thus, a particularly preferred cleavage process of the present invention is one wherein a compound of the formula 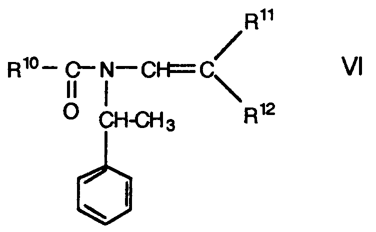
is cleaved with trifluoroacetic acid. In the above formula, R¹⁰, R¹¹ and R¹² in the broadest sense represent groups which are not reactive with trifluoroacetic acid. In a more preferred sense, those groups represent alkyl, cycloalkyl or aryl groups which may be substituted with groups which are not reactive with trifluoroacetic acid. Still more preferred is the above process wherein R¹⁰, R¹¹ and R¹² combine to complete a heterocyclic system comprising from one to about four rings which may be substituted with groups which are not reactive with trifluoroacetic acid.

Such non-reactive groups include, for example, alkyl, halo, alkanoyl, alkoxycarbonyl, aroyl, alkoxy, and like groups. In a most preferred aspect, R¹⁰, R¹¹ and R¹² combine to complete a heterocyclic system which is benzoquinoline, in any of its oxidation states which correspond to the above structure, and substituted in any non-reactive manner.

A preferred aspect of the heteroannulation process is an embodiment of it in which R¹ represents a chiral auxiliary, such as amino acid derivatives including esters, amides and alcohols, and particularly derivatives of the 1-phenylethyl group, wherein the phenyl group may be substituted with non-interfering groups such as alkyl, halo, amino, alkoxy and the like. The concept of chiral auxiliaries is well understood by organic chemists and their use here is typical of their use in known organic synthesis. The particularly preferred chiral auxiliary is 1-phenylethyl.

It will be understood that the use of an R¹ group which is a chiral auxiliary is particularly advantageous when preparing a product which is optically active and is expected to exist as a racemic mixture. Ordinarily, in pharmaceutical chemistry, it is desirable or necessary to obtain a single optical isomer for use. It will be understood that such a racemic product mixture, having a chiral auxiliary at the R¹ position, is readily separated into its individual optical isomers by conventional procedures which are well understood by organic chemists.

A further particularly preferred embodiment of the heteroannulation process is the use of it to prepare a compound of the formula 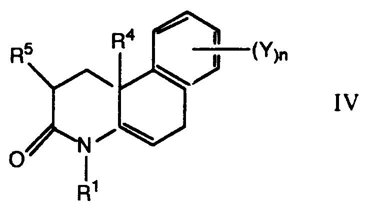
wherein
R¹ is as described above, and most preferably is 1-phenylethyl;
R⁵ is hydrogen or C₁-C₄ alkyl;
R⁴ is methyl;
and Y is hydrogen, halo, cyano, trifluoromethyl, C₁-C₃ alkyl, alkoxycarbonyl, aminocarbonyl, alkylaminocarbonyl, dialkylaminocarbonyl, alkylsulfonyl, alkylsulfinyl, C₁-C₃ alkoxy, amino, C₁-C₃ alkyl- or dialkylamino, C₁-C₃ alkylthio or C₁-C₃ haloalkyl;
and n is 1 or 2.

The synthesis of the above compounds is easily carried out by reacting an enamine of the formula 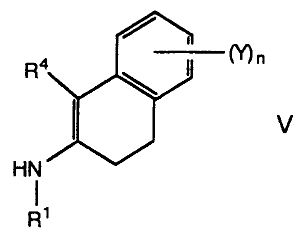
with an acryloyl derivative of the formula

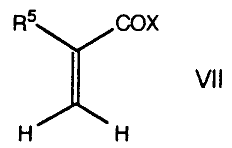

The above particularly preferred products are intermediates in the preparation of inhibitors of the enzyme 5α-reductase, which compounds are disclosed in EP-A-532190, 1991, which are also useful for treating a variety of disorders which have been linked to that activity in mammals. Among those disorders are benign prostatic hyperplasia, male pattern baldness, acne, hirsutism and prostatic cancer. The compounds are used to treat such disorders by administration to a patient in need of such treatment, either therapeutically or prophylactically, by any of the typical pharmaceutical routes of administration including oral, subcutaneous, intravenous, topical, intramuscular and intranasal.

Still further, the heteroannulation process of the present invention is conveniently used to prepare many other biologically active molecules. For example, the following group of such compounds is mentioned for the convenience of the skilled reader. It will be understood that some of the following compounds are made by multiple-step processes, in which additional steps after the heteroannulation are required. In some cases, while the following compounds are all known to ordinarily skilled pharmaceutical chemists, a reference is mentioned as a source of further information about the compound.

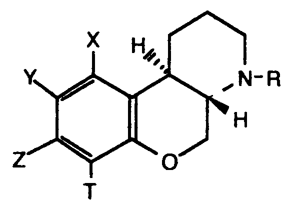

| | X | Y | Z | T | R |
|---|---|---|---|---|---|
| CGS 15855A | H | OH | H | H | n-Pr |
| CGS 15873A | H | H | H | OH | n-Pr |
| CGS 19845 | Me | H | H | OH | Me |
| CGS 18102 | OMe | H | H | H | n-Pr |

J. Med. Chem., 32, 720-27 (1989)

### Hoechst 833

[2S-(2α,7β,7aβ,14β,14aα)]-1H-pyrrole-2-carboxylic acid, dodecahydro-11-oxo-7,14-methano-2H,6H-dipyrido [1,2-a:1',2'-e][1,5]diazocin-2-yl ester

### Hydroxylupanine-13

[7S-(7α,7aβ,9β,14α,14aα)]-dodecahydro-9-hydroxy-7,14-methano-4H,6H-dipyrido[1,2-a:1',2'-e][1,5]diazocin-4-one

### L-636028

N,N-diethylhexadecahydro-1-methyl-2-oxo-1H-indeno[5,4-f]quinoline-7-carboxamide
Larson, Karen A; King, Michael L., Biotechnol. Prog., 2(2), pp 73-82 (1986)

### L-637146

5α-4-methyl-4-azapregnane-3,20-dione,
U.S. Patent No. 4,377,584

### LL-AB-664

[3aS-(3aα,7α,7aβ)]-2-[[4-O-(aminocarbonyl)-2-deoxy-2-[[[(iminomethyl)amino]acetyl]methylamino]-β-D-gulopyranosyl]amino]-1,3a,5,6,7,7a-hexahydro-7-hydroxy-5-methyl- 4H-imidazo[4,5-c]pyridin-4-one Kawakami, Yoshiyuki; Yamasaki, Kazuo; Nakamura, Shoshiro, J. Antibiot., 34(7), pp 921-2 (1981)

### Lupanine

7S-(7α,7aβ,14α,14aα)]-dodecahydro-7,14-methano-4H,6H-dipyrido[1,2-a:1',2'-e][1,5]diazocin-4-one

### Matrine

Matridin-15-one
U.S. Patent No. 5,041,450

### MK-771

[2S-[2R*[R*(R*)]]]-N-[2-[4-(aminocarbonyl)-3-thiazolidinyl]-1-(1H-imidazol-4-ylmethyl)-2-oxoethyl]-6-oxo-2-piperidinecarboxamide

### PK10157

4-[2-(1H-indol-3-yl)ethyl]-2-piperidinone
Toupin, L.; Caille, G.; Vezina, M.; Demontigny, C.; Tawashi, M., Biopharm. Drug Dispos., 8(6), pp 507-17 (1987)

### Streptothrin

Streptothricin
U.S. Patent No. 3,876,778

### Streptothrin-E

3aS-[2[R*(R*)],3aα,7α,7aβ]]-2-[[4-O-(aminocarbonyl)-2-[[3-amino-6-[(3,6-diamino-1-oxohexyl)amino]-1-oxohexyl]amino]-2-deoxy-β-D-gulopyranosyl]amino]-1,3a,5,6,7,7a-hexahydro-7-hydroxy- 4H-imidazo[4,5-c]pyridin-4-one

### Streptothrin-F

3aS-[2(R*),3aα,7α,7aβ]]-2-[[4-O-(aminocarbonyl)-2-deoxy-2-[(3,6-diamino-1-oxohexyl)amino]-β-D-gulopyranosyl]amino]-1,3a,5,6,7,7a-hexahydro-7-hydroxy-4H-imidazo[4,5-c]pyridin-4-one
U.S. Patent No. 3,962,426

### WB-CPI

3-hydroxy-2-oxo-3-phenyl-1-piperidinecarboxylic acid
Brouillette, Wayne J.; Grunewald, Gary L., J. Med. Chem., 27(2), pp 202-6 (1984)

### Glorin

N-(2-oxo-3-piperidinyl)-N₂-(1-oxopropyl)-L-glutamine, ethyl ester
Ball, Jonathan B.; Craik, David J..; Alewood, Paul F.; Morrison, Stuart; Andrews, Peter R.; Nicholls, Ian A., Aust. J. Chem., 42(12), pp 2171-80 (1989)

### CP-9337AH

1-hydroxy-3-(3-3H)(4-chlorophenyl)-2-piperidone Bovy, P.; Callaert, M.; Gillet, C.; Decker, J.M. de; Winand, M., J. Labelled Compd. Radiopharm.(23, No. 6., 577-85, 1986)

### AN-201 I/AN-201 II/AN-201 III

Ando, T.; Miyashiro, S.; Hirayama, K.; Kida, T.; Shibai, H.; Murai, A., J. Antibiot. (40, No. 8, 1140-45, 1987)

### Albothricin

3aS-[2(R*),3aα,7aβ]]-2-[[4-O-(aminocarbonyl)-2-deoxy-2-[(3,6-diamino-1-oxohexyl)amino]-β-D-gulopyranosyl]amino]-1,3a,5,6,7,7a-hexahydro-5-methyl-4H-imidazo[4,5-c]pyridin-4-one
Ohba, Kazunori; Nakayama, Hiroshi; Furihata, Kazuo; Furihata, Keiko; Shimazu, Akira; Seto, Haruo; Otake, Noboru; Yang, Zhaozhong; Xu, Lisha; Xu, Wensi, J. Antibiot., 39(6), pp 872-5 (1986)

### 4-MA

5α,17β-N,N-diethyl-4-methyl-3-oxo-4-azaandrostane-17-carboxamide
U.S. Patent No. 4,377,584

### A-37812

Hunt, A.H.; Hamill, R.L.; DeBoer, J.R.; Presti, E.A., J. Antibiot. (38, No. 8, 987-92, 1985)

### Zoanthamide

[4'aα,5'β,6'aβ,7'β,7'aβ,11'(2S*,4S*),12'aα,12'bβ]-(+)-1',4'a,5',8',9',11',12'a,12'b-octahydro-2',5',7'a,12'a-tetramethyl-11'-[(tetrahydro-4-methyl-5- oxo-2-furanyl)methyl]-,spiro[furan-3(2H),7'(4'H)-naphtho[2,1-g]quinoline]-4',5,6',10'(4H,6'aH,7'aH)-tetrone
Atta-ur-Rahman; Alvi, K. Ahmed; Abbas, S. Ali; Choudhary, M. Iqbal; Clardy, Jon, Tetrahedron Lett., 30(49), 6825-8 (1989)

### S-26191

5-hydroxy-N-[(6-oxo-2-piperidinyl)methyl]-2-(2,2,2-trifluoroethoxy)-benzamide
U.S. Patent No. 4,555,573

### S-321328

[6aR-[6aα,7α(R*),9aβ,9bα]]-2,3,4,6,6a,7,8,9,9a,9b-decahydro-gamma,6a-dimethyl-3-oxo-1H-cyclopenta[f]-quinoline-7-butanoic acid
Hayakawa, Shohei; Fujiwara, Takashi, Biochem. J., 162(2), pp 387-97 (1977)

### Posatirelin

(S)-N-[(6-oxo-2-piperidinyl)carbonyl]-L-leucyl-L-prolinamide
Ger. Offen. DE 3024256

The following examples illustrate the processes of the present invention, and are intended to improve the reader's comprehension of the invention and the manner in which the processes are carried out. In the following examples, the usual organic chemical conventions are followed. The abbreviation FDMS refers to field desorption mass spectroscopy. The term HPLC refers to high pressure liquid chromatography, and HRMS refers to high resolution mass spectroscopy.

### Example 1

### (+)-(4aR)-(10bR)-8-chloro-1,2,3,4,4a,5,6,10b-octahydrobenzo[f]quinoline-3-one

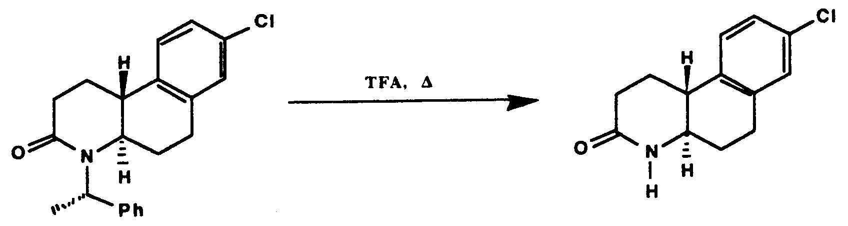

A mixture of the above intermediate (1.0 equiv., 4.09 mmol., 1.39 g) and trifluoroacetic acid (35 ml) were combined in a round bottom flask. The stirred mixture was heated at reflux for 2 hrs. The cooled reaction mixture was concentrated in vacuo, taken up in dichloromethane and washed with a saturated aqueous solution of sodium bicarbonate (1x). The layers were separated and the aqueous layer was back extracted with dichloromethane (1x). The combined organics were washed with brine (1x), dried (Na₂SO₄), and concentrated in vacuo, to give a white solid. Recrystallization from ethyl acetate gave 0.81g, 84% yield of the title compound as colorless needles. mp 241.5-242°. FDMS: m/e = 235. α[D]₅₈₉ = 32.61 (*c* = 1.0, THF).

| | Theory | Found |
|---|---|---|
| C | 66.24 | 66.35 |
| H | 5.99 | 6.10 |
| N | 5.94 | 5.83 |

### Example 2

### (-)-(10bR)-8-chloro-10b-ethoxycarbonyl-4-(S-α-methylbenzyl)-1,2,3,4,6,10b-hexahydrobenzo[f]quinoline-3-one

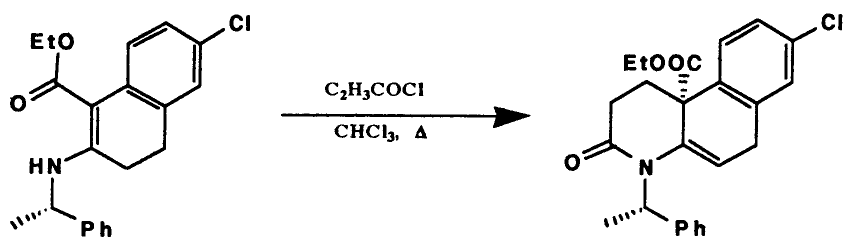

To a stirred solution of (S)-(-)-6-chloro-1-ethoxycarbonyl-2-(α-methylbenzylamino)-3,4-dihydronaphthalene (1.0 equiv., 5.96 mmol., 1.91 g) in chloroform (30 ml) was added acryloyl chloride (1.2 equiv., 7.15 mmol., 0.49 ml). The mixture was heated at reflux under a nitrogen atmosphere with stirring for 1.5 hrs. The cooled reaction mixture was concentrated in vacuo and subjected to flash chromatography on silica gel (elution with 15% ethyl acetate/hexanes) to give 1.99 g, 81 % yield of the title compound as a colorless foam. HPLC analysis (reverse phase, C₁₈ ,230nm, mobile phase: 60%
acetonitrile/40% 0.5% ammonium acetate buffer) revealed a 35:1 mixture of diastereoisomers. FDMS: m/e = 410. α[D]₅₈₉ = -3.20 (*c* = 1.0, CHCl₃).

| | Theory | Found |
|---|---|---|
| C | 70.15 | 70.14 |
| H | 6.13 | 5.91 |
| N | 3.41 | 3.52 |

### Example 3

### (+)-(10bS)-10b-methyl-4-(S-α-methylbenzyl)-1,2,3,4,6,10b-hexahydrobenzo[f]quinoline-3-one

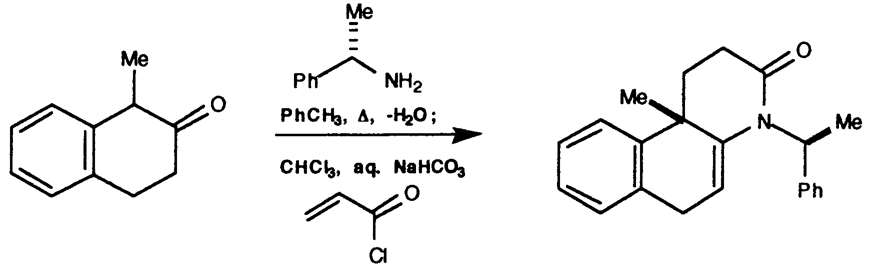

To a stirred solution of 1-methyl-2-tetralone (1.0 equiv., 56.0 mmol., 9.0 g) in toluene (100 mL) was added (S)-(-)-α-methylbenzylamine (1.05 equiv., 59.0 mmol, 7.6 mL). The mixture was heated to reflux for 1 hour with azeotropic removal of water (Dean-Stark). The solution was cooled to ambient temperature and concentrated under reduced pressure to afford crude enamine, 1-methyl-2-(α-methylbenzylamino)-3,4-dihydronaphthalene, as a yellow oil. The oil was dissolved in CHCl₃ (100 mL) and treated with saturated aqueous NaHCO₃ (100 mL). The mixture was vigorously stirred and treated with acryloyl chloride (1.0 equiv., 56.0 mmol, 4.5 mL) in one portion. After 1 minute, the layers were separated and the aqueous layer back extracted with 2X 100 mL of CHCl₃. The combined organic phases were dried over anhydrous Na₂SO₄ and concentrated to afford a yellow glass. This crude product was purified by flash chromatography on silica (25% ethyl acetate/hexanes as eluent) to afford 13.4 g of title compound as a yellow solid. Crystallization from ethyl acetate/hexanes gave an analytical sample m/e= 317. α[D]₅₈₉= 35.88° (c=1.0, CHCl₃). Single crystal x-ray diffraction analysis confirmed stereochemistry.

| | Theory | Found |
|---|---|---|
| C | 83.24 | 82.97 |
| H | 7.30 | 7.40 |
| N | 4.41 | 4.46 |

### Example 4

### (8aR)-4-(S-α-methylbenzyl)-1,2,3,4,6,7,8,8a-octahydroquinoline-3,8-dione

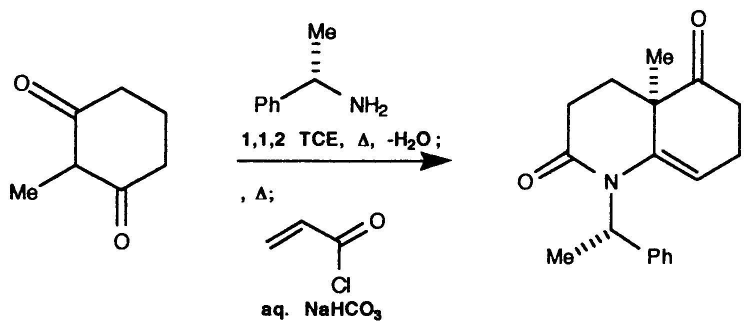

To a stirred solution of 2-methyl-cyclohexane-1,3-dione (1.0 equiv., 1.44 mmol., 332 mg) in 1,1,2-trichloroethane (20 mL) was added (S)-(-)-α-methylbenzylamine (1.05 equiv., 1. 51 mmol., 0.2 mL). The mixture was heated to reflux for 1 hour with azeotropic removal of water (Dean-Stark) to prepare the enamine, 1-(α-methylbenzylamino)-2-methyl-1-cyclohexane-3-one. The solution was cooled to ambient temperature and treated with acryloyl chloride (1.5 equiv., 2.17 mmol., 176 µL) in one portion. After 2 hours, the solution was cooled to room temperature and treated with saturated aqueous NaHCO₃ (20 mL). The layers were separated and the aqueous layer back extracted with 2X 100 mL of CHCl₃. The combined organic phases were dried over anhydrous Na₂SO₄ and concentrated to afford a yellow glass. This crude product was purified by flash chromatography on silica (25% ethyl acetate/hexanes as eluent) to afford 289 mg of title compound as a yellow solid. Crystallization from ethyl acetate/hexanes gave an analytical sample (mp 138°). m/e= . α[D]₅₈₉= +7.08° (c=1.0, CHCl₃).

| | Theory | Found |
|---|---|---|
| C | 76.30 | 76.59 |
| H | 7.47 | 7.65 |
| N | 4.94 | 5.13 |

### Preparation 1

### 1-benzoyl-5-methyl-4-(S-α-methylbenzyl)-1,2,2a,3-tetrahydrobenz[cd]indole

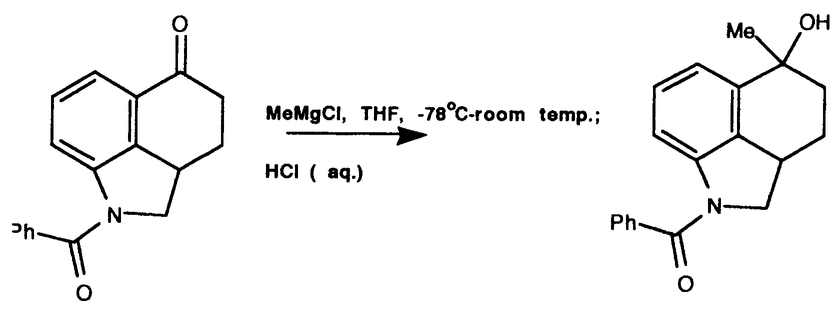

To a stirred, cooled (-78°C) solution of Kornfeld's ketone (1.0 equiv., 60 mmol., 16.7 g) in THF (500 mL) was added methylmagnesium chloride (3.0 M in THF, 1.1 equiv., 66.0 mmol., 22 mL) dropwise. The solution was gradually warmed to room temperature over 2 hours and stirred an additional 2 hours. The reaction was quenched by slow addition of water (50 mL) followed by 1N HCl solution (70 mL). The mixture was extracted with ethyl acetate and the organic layers dried over Na₂SO₄ and concentrated to afford crude alcohols contaminated by a small amount of starting ketone (ca. 10%). Purification by preparative HPLC on silica (25% ethyl acetate/hexanes as eluent) afforded 13.02 g of 1-benzoyl-5-hydroxy-5-methyl-1,2,2a,3,4,5-hexahydrobenz[c,d]indole as a mixture of diastereomers.

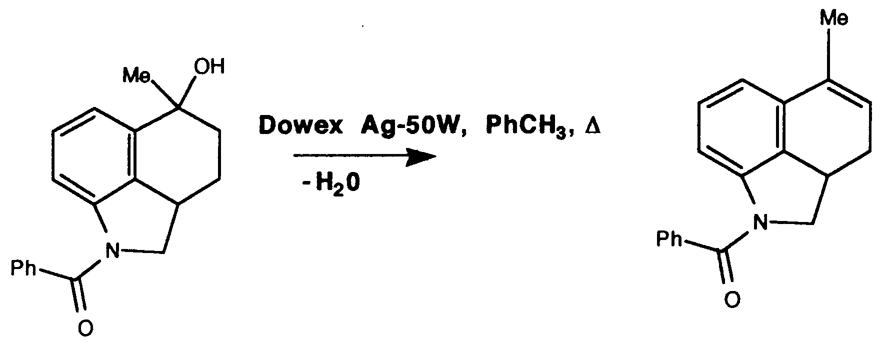

Dowex AG-50W resin (3.9 g) was placed in a flask equipped with stirrer, nitrogen inlet and Dean-Stark apparatus, with toluene (500 mL). The mixture was heated to reflux for 30 minutes and cooled to ambient temperature. The above prepared alcohol (12.5 g, 42.6 mmol.) in 200 mL of toluene was added and the mixture further refluxed with azeotropic removal of water for 10 hrs. An additional 4.0 g of Dowex AG-50W resin was added and the mixture further refluxed for 1 hr. The reaction mixture was cooled to ambient temperature and filtered. Concentration afforded the product olefin, 1-benzoyl-5-methyl-1,2,2a,3-tetrahydrobenz[c,d]indole, (8.97 g) as a pale yellow crystalline solid, used without further purification.

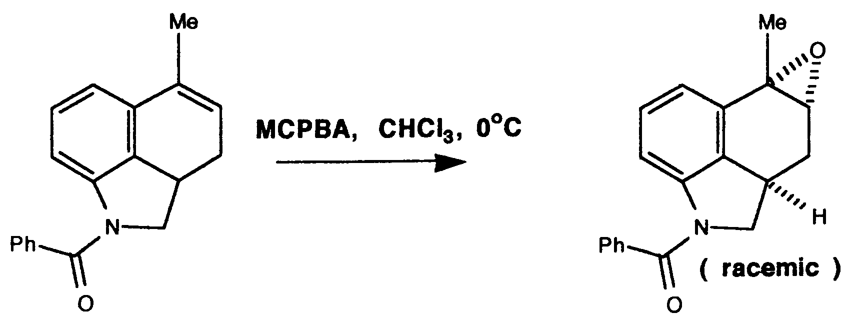

To a stirred, cooled (0°C) solution of the above olefin (1.0 equiv., 32.3 mmol., 8.9 g) in CHCl₃ (250 mL) was added meta-chloroperbenzoic acid (55-60% , 1.08 equiv., 35 mmol., 12 g) in small portions, keeping the reaction temperature < 5°C. After complete addition, the mixture was further stirred for 1 hour and poured into saturated aqueous NaHCO₃ solution (250 mL), The layers were separated and the aqueous layer back extracted with CHCl₃ (250 mL). The combined organic layers were washed with 1N sodium thiosulfate solution (100 mL) and dried over Na₂SO₄. Removal of solvent under reduced pressure afforded 8.45 g of product epoxide, 1-benzoyl-5-methyl-4,5-epoxy-1,2,2a,3,4,5-hexahydrobenz[c,d]indole, as a white crystalline solid. The crude epoxide was again used without further purification.

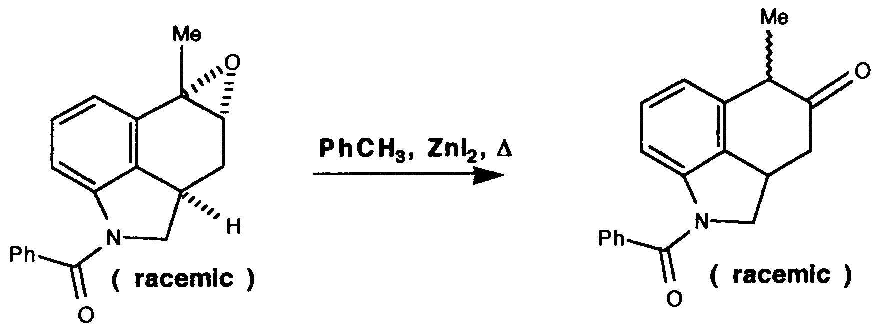

A suspension of zinc iodide (0.12 equiv., 3.1 mmol., 1.0 g) was heated to reflux in toluene (200 mL) under nitrogen for 30 min. The solution was cooled to 90°C and maintained at that temperature with an oil bath. To the solution was added the above epoxide (1.0 equiv., 28.9 mmol., 8.45 g) in toluene (50 mL) dropwise over 40 min. After complete addition, the mixture was further heated for 30 minutes and cooled to ambient temperature. The mixture was filtered through celite, washing the filter cake with toluene (500 mL). The filtrates were concentrated under reduced pressure to afford product ketones as a brown oil. Purification by flash chromatography on silica (25% ethyl acetate/hexanes as eluent) afforded 8.4 g of product, 1-benzoyl-5-methyl-4-oxo-1,2,2a,3,4,5-hexahydrobenz[c,d]indole, as a mixture of diastereomers.

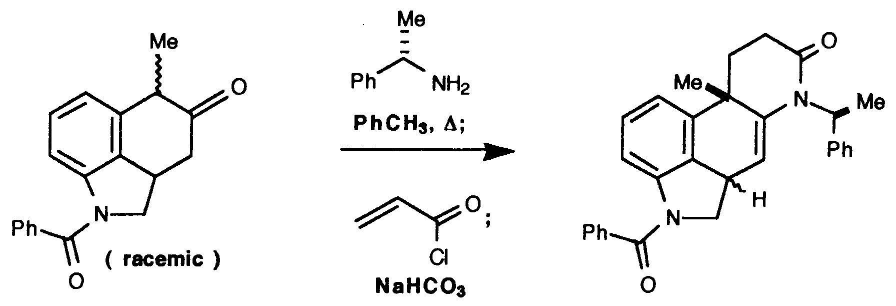

To a stirred solution of the ketone above (1.0 equiv., 28.9 mmol., 8.4 g) in toluene (200 mL) was added (S)-(-)-α-methylbenzylamine (1.05 equiv., 30.0 mmol., 3.9 mL). The mixture was heated to reflux for 14 hours with azeotropic removal of water (Dean-Stark). The solution was cooled to ambient temperature and concentrated under reduced pressure to afford the desired crude enamine as a brown foam.

### Example 5

### (-)-1,4,5a,6,7,10b-hexahydro-4(S-α-methylbenzyl)-7-benzoyl-10b-(S-methylindolo)[fg]-quinoline-3(2H)-one

The enamine of Preparation 1 was dissolved in CHCl₃ (200 mL) and was vigorously stirred and treated with acryloyl chloride (1.05 equiv., 30.0 mmol., 2.4 mL) in one portion. After 10 minutes, the reaction mixture was treated with saturated aqueous NaHCO₃ solution (200 mL). The layers were separated and the aqueous layer back extracted with 2X 100 mL of CHCl₃. The combined organic phases were dried over anhydrous Na₂SO₄ and concentrated to afford a brown glass. This crude product was purified by flash chromatography on silica (35% ethyl acetate/hexanes as eluent) to afford 3.7 g and 3.4 g of diastereomeric products. The minor product was crystallized from ethyl acetate/hexanes to afford a crystalline solid (mp 262°). m/e= 448. α[D]₅₈₉=-84.55(C=1, CHCl₃).

| | Theory | Found |
|---|---|---|
| C | 80.33 | 80.06 |
| H | 6.29 | 6.44 |
| N | 6.25 | 6.41 |

### Example 6

### (+)-(4aS)-4a-ethoxycarbonyl-1-(α-methylbenzyl)-2,3,4,4a,5,6,7-heptahydroquinoline-2-one

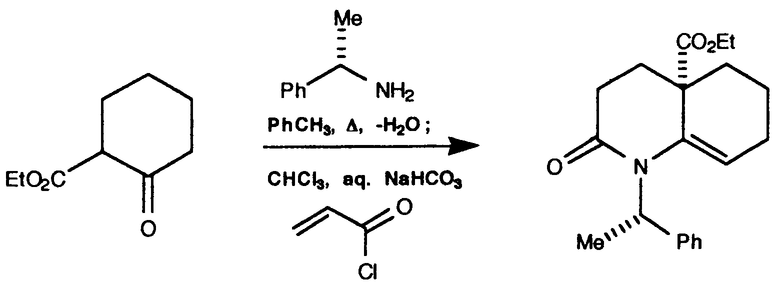

To a stirred solution of carboethoxycyclohexanone (1.0 equiv., 50.0 mmol., 7.94 g) in toluene (200 mL) was added (S)-(-)-α-methylbenzylamine (1.04 equiv., 52.0 mmol., 6.7 mL). The mixture was heated to reflux for 10 hours with azeotropic removal of water (Dean-Stark). The solution was cooled to ambient temperature and concentrated under reduced pressure to afford crude enamine, (S)-2-ethoxycarbonyl-1-(α-methylbenzylamino)-2-cyclohexene, as a yellow oil. The oil was dissolved in CHCl₃ (150 mL) and treated with saturated aqueous NaHCO₃ (150 mL). The mixture was vigorously stirred and treated with acryloyl chloride (1.0 equiv., 56.0 mmol., 4.5 mL) in one portion. After 1 hour, the layers were separated and the aqueous layer back extracted with 2X 100 mL of CHCl₃. The combined organic phases were dried over anhydrous Na₂SO₄ and concentrated to afford a yellow glass. This crude product was purified by flash chromatography on silica (25% ethyl acetate/hexanes as eluent) to afford 5.54 g of title compound as a yellow solid. Crystallization from ethyl acetate/hexanes gave an analytical sample (mp 76.5°C). m/e= 328. α[D]₅₈₉= +122.75° (c=1.0, CHCl₃).

| | Theory | Found |
|---|---|---|
| C | 73.36 | 73.53 |
| H | 7.70 | 7.71 |
| N | 4.28 | 4.49 |

### Example 7

### (-)-(10bS)-8,9-dichloro-10b-methyl-4-(S-α-methylbenzyl)-1,2,3,4,6,10b-hexahydrobenzo[f]quinoline-3-one

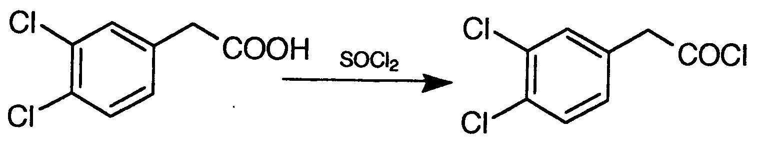

In a 1L one-neck flask fitted with magnetic stirrer and drying tube was dissolved 3,4-dichlorophenylacetic acid (49.6 g, 242 mmol.) in thionyl chloride (200 mL, 2.74 mmol.) at ambient temperature. The solution was stirred for 12 H and concentrated under reduced pressure to afford 3,4-dichlorophenylacetyl chloride (54.1 g) as a pale oil which was used without further purification.

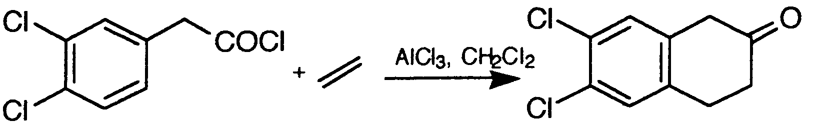

In a 1L, 3-neck flask fitted with a thermometer, N₂ inlet and mechanical stirrer was slurried aluminum chloride (65.1 g, 489 mmol.) in dichloromethane (750 mL). The suspension was cooled to -75°C and treated with a solution of acid chloride (54.1 g, 242 mmol.) prepared above in dichloromethane (250 mL) added dropwise over 1 H. The mixture was warmed to -20°C and treated with ethylene gas, added through a gas dispersion tube for 15 min. Over this addition period, the reaction temperature increased to 10°C. The addition was ceased and the cooling bath removed and the reaction mixture stirred at ambient temperature for 3 H. The mixture cooled to 0°C and treated with ice (500 g). The mixture was stirred until all solid material had dissolved, The phases were separated and the organic phase washed with 1 N HCl, water, and saturated sodium bicarbonate solution, The solution was dried over sodium sulfate and concentrated under reduced pressure. The crude brown solid was dissolved in ethyl acetate/hexanes and treated with decolorizing carbon and filtered. Crystallization afforded 6,7-dichloro-2-tetralone (19,3 g) as a light brown solid. mp. 97-100°C.

| | Theory | Found |
|---|---|---|
| C | 55.84 | 55.86 |
| H | 3.74 | 3.77 |
| N | - | - |

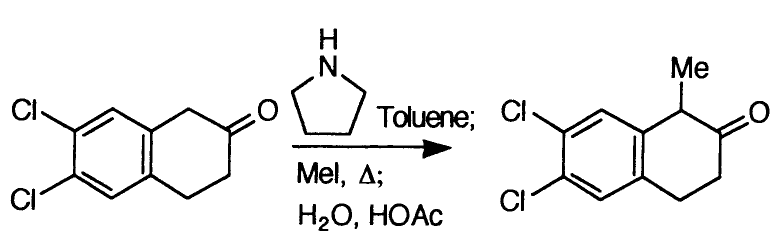

In a 1 L flask fitted with reflux condenser, N₂ inlet, Dean-Stark trap and magnetic stirrer was dissolved 6,7-dichloro-2-tetralone (19.3 g, 89.7 mmol) prepared above in toluene (500 mL). Pyrrolidine (11.2 mL, 135 mmol) was added and the solution heated to reflux with azeotropic removal of water for 80 min. The solution was cooled and the solvent removed under reduced pressure. The crude enamine was dissolved in dioxane (250 mL) and treated with methyl iodide (31.3 mL, 502 mmol). The solution was heated to reflux for 14 H. The reaction mixture was cooled to ambient temperature and concentrated under reduced pressure. The brown solid was redissolved in dioxane (250 mL) and treated with water (47 mL) and acetic acid (1.9 mL). The mixture was heated to reflux for 3.5 H. The mixture was cooled to ambient temperature and partitioned between ethyl acetate and water. The organic layer was washed with water, 1 N HCl, saturated sodium bicarbonate solution and brine and dried over sodium sulfate. The crude product was isolated by removal of solvent under reduced pressure and purified by preparative HPLC, eluting with 7.5% ethyl acetate/toluene. Fractions containing product were pooled and concentrated under reduced pressure to afford 1-methyl-6,7-dichloro-2-tetralone (12.3 g) as a yellow oil which solidified on standing. mp. 65-67°C.

| | Theory | Found |
|---|---|---|
| C | 57.67 | 57.70 |
| H | 4.40 | 4.40 |
| N | - | - |

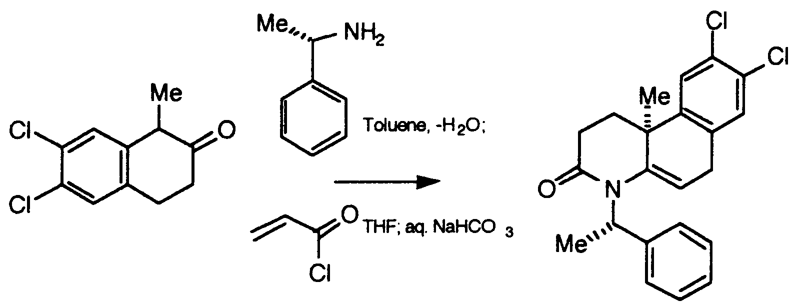

In a 250 mL flask fitted with a Dean-Stark trap, magnetic stirrer and reflux condenser was dissolved 1-methyl-6,7-dichloro-2-tetralone (2.0 g, 8.7 mmol) in toluene (150 mL). S(-)-phenethylamine (1.2 mL, 9.6 mmol) was added and the solution heated to reflux with azeotropic removal of water for 12 H. The solution was cooled to ambient temperature and concentrated under reduced pressure. The crude enamine was dissolved in THF (100 mL) and treated with acryloyl chloride (780 µL, 9.6 mmol) in a single portion. The solution was stirred at ambient temperature for 10 min. and quenched by addition of aqueous sodium bicarbonate solution. The layers were separated and the organic phase dried over magnesium sulfate and concentrated under reduced pressure. Flash chromatography on silica gel, eluting with 25% ethyl acetate/hexanes afforded product (2.3 g) as a colorless foam. m/e 385.

| | Theory | Found |
|---|---|---|
| C | 68.4 | 68.39 |
| H | 5.48 | 5.35 |
| N | 3.63 | 3.56 |

### Example 8

### (-)-(4aS)-(10bS)-8,9-dichloro-10b-methyl-1,2,3,4,4a,5,6,10b-octahydrobenzo[f]quinoline-3-one

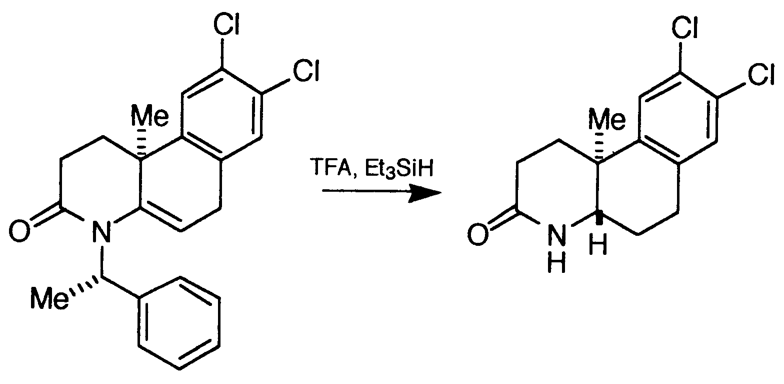

In a 250 mL flask fitted with N2 inlet, magnetic stirrer and reflux condenser was dissolved enamide (2.8 g, 7.2 mmol) previously prepared in trifluoroacetic acid (10 mL). To the bright yellow solution was added triethylsilane (20 mL) and the mixture heated to reflux for 4.5 H. The solution was cooled to ambient temperature and concentrated under reduced pressure. The crude product was dissolved in ethyl acetate and washed with saturated sodium bicarbonate solution, dried over sodium sulfate and concentrated under reduced pressure. The residue was triturated with pentane (2X) to afford a solid. Recrystallization from ethyl acetate/hexanes afforded product (730 mg) as white crystals. mp = 196-198°C, m/e 283. a[D]₅₈₉= -111.1° (c=1.0, CHCl₃), a[D]₃₆₅= -378.7° (c=1.0, CHCl₃).

| | Theory | Found |
|---|---|---|
| C | 59.17 | 58.91 |
| H | 5.32 | 5.22 |
| N | 4.93 | 4.84 |

### Example 9

### (+)-(4aR)-(10bS)-8,9-dichloro-10b-methyl-1,2,3,4,4a,5,6,10b-octahydrobenzo[f]quinoline-3-one

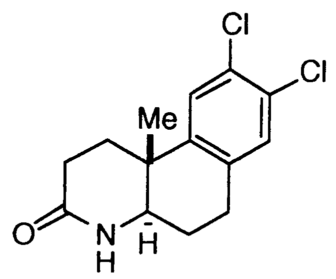

Product was prepared in the manner of Example 7, step 4, and Example 8, except using R(+)-phenethylamine in place of S(-)-phenethylamine. mp = 195-198°C, m/e 283. a[D]₅₈₉= 97.1° (c=1.0, CHCl₃), a[D]₃₆₅= 329.8° (c=1.0, CHCl₃ ).

| | Theory | Found |
|---|---|---|
| C | 59.17 | 59.14 |
| H | 5.32 | 5.13 |
| N | 4.93 | 4.77 |

### Example 10

### (4aR)-7-acetamido-10b-methyl-4-(R-α-methylbenzyl)-1,2,3,4-tetrahydrobenzo[f]-1-pyrindine-3-one

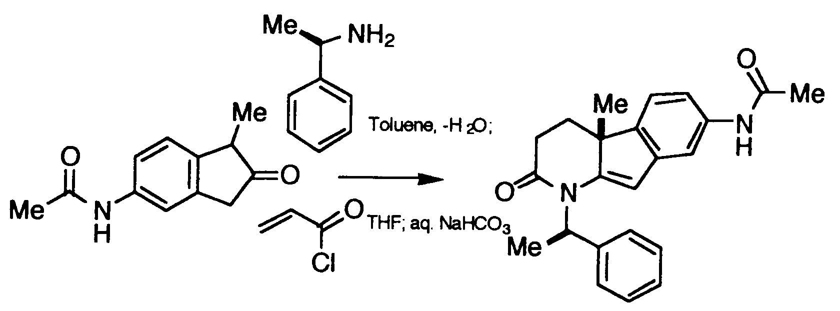

In a 250 mL flask fitted with a Dean-Stark trap, magnetic stirrer and reflux condenser was dissolved 1-methyl-5-acetamido-2-indanone (1.7 g, 8.4 mmol) in toluene (150 mL). R(+)-phenethylamine (1.2 mL, 9.6 mmol) was added and the solution heated to reflux with azeotropic removal of water for 12 H. The solution was cooled to ambient temperature and concentrated under reduced pressure. The crude enamine was dissolved in THF (100 mL) and treated with acryloyl chloride (747 µL, 9.2 mmol) in a single portion. The solution was stirred at ambient temperature for 10 min. and quenched by addition of aqueous sodium bicarbonate solution. The layers were separated and the organic phase dried over magnesium sulfate and concentrated under reduced pressure. Flash chromatography on silica gel, eluting with 3% methanol/dichloromethane afforded product (1.35 g) as a foam. m/e 360.

| | Theory | Found |
|---|---|---|
| C | 76.64 | 76.80 |
| H | 6.71 | 6.78 |
| N | 7.77 | 7.57 |

### Example 11

### (-)-(10bS)-8-bromo-10b-methyl-4-(S-α-methylbenzyl)-1,2,3,4,6,10b-hexahydrobenzo[f]quinoline-3-one

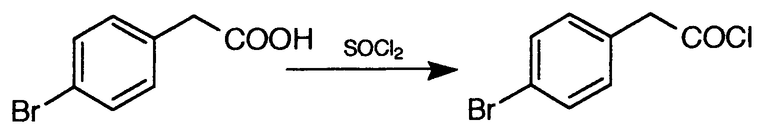

In a 1L one-neck flask fitted with magnetic stirrer and drying tube was dissolved 4-bromophenylacetic acid (100 g, 465 mmol) in thionyl chloride (100 mL, 1.37 mol) at ambient temperature. The solution was stirred for 12 H and concentrated under reduced pressure to afford 4-bromophenylacetyl chloride (108 g) as a pale oil which was used without further purification.

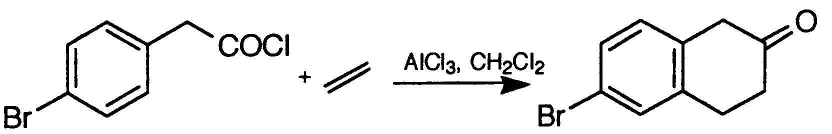

In a 3L, 3-neck flask fitted with a thermometer, N₂ inlet and mechanical stirrer was slurried aluminum chloride (123.9 g, 930 mmol) in dichloromethane (800 mL). The suspension was cooled to -75°C and treated with a solution of acid chloride (108 g, 465 mmol) prepared above in dichloromethane (200 mL) added dropwise over 1 H. The mixture was warmed to -20°C and treated with ethylene gas, added through a gas dispersion tube for 15 min. Over this addition period, the reaction temperature increased to 10°C. The addition was ceased and the cooling bath removed and the reaction mixture stirred at ambient temperature for 3 H. The mixture cooled to 0°C and treated with ice (500 g). The mixture was stirred until all solid material had dissolved. The phases were separated and the organic phase washed with 1 N HCl, water, and saturated sodium bicarbonate solution. The solution was dried over sodium sulfate and concentrated under reduced pressure. The crude product (107.1 g) was used without further purification.

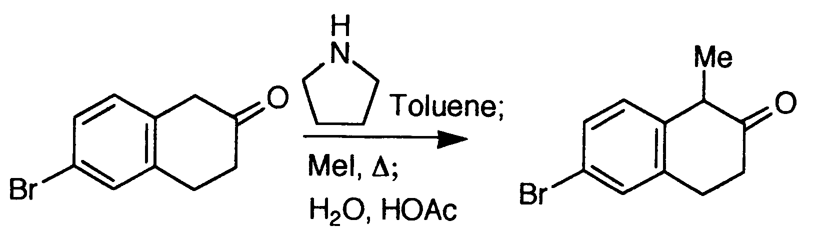

In a 1 L flask fitted with reflux condenser, N₂ inlet, Dean-Stark trap and magnetic stirrer was dissolved 7-bromo-2-tetralone (104.7 g, 465 mmol) prepared above in toluene (1500 mL). Pyrrolidine (58.2 mL, 697 mmol) was added and the solution heated to reflux with azeotropic removal of water for 4 H. The solution was cooled and the solvent removed under reduced pressure. The crude enamine was dissolved in dioxane (700 mL) and treated with methyl iodide (174 mL, 2.8 mol). The solution was heated to reflux for 14 H. The reaction mixture was cooled to ambient temperature and concentrated under reduced pressure. The brown solid was redissolved in dioxane (250 mL) and treated with water (47 mL) and acetic acid (1.9 mL). The mixture was heated to reflux for 3.5 H. The mixture was cooled to ambient temperature and partitioned between ethyl acetate and water. The organic layer was washed with water, 1 N HCl, saturated sodium bicarbonate solution and brine and dried over sodium sulfate. The crude product was isolated by removal of solvent under reduced pressure and purified by preparative HPLC, eluting with 5 % ethyl acetate/toluene. Fractions containing product were poled and concentrated under reduced pressure to afford 1-methyl-7-bromo-2-tetralone (70.5 g) as a brown oil.

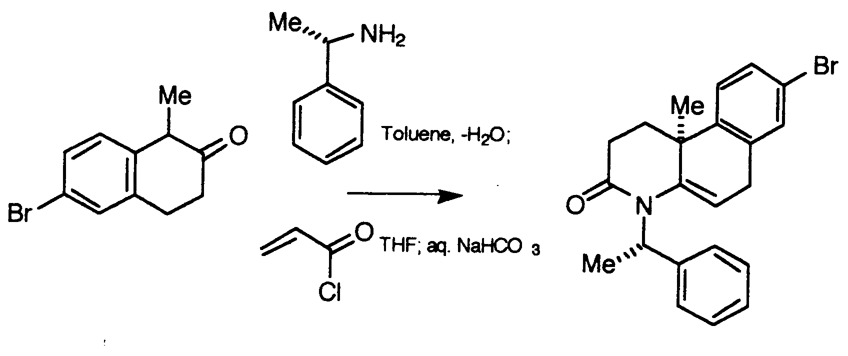

In a 500 mL flask fitted with a Dean-Stark trap, magnetic stirrer and reflux condenser was dissolved 1-methyl-7-bromo-2-tetralone (16.0 g, 66.9 mmol) in toluene (300 mL). S(-)-phenethylamine (8.63 mL, 66.9 mmol) was added and the solution heated to reflux with azeotropic removal of water for 12 H. The solution was cooled to ambient temperature and concentrated under reduced pressure. The crude enamine was dissolved in THF (200 mL) and treated with acryloyl chloride (5.7 mL, 70.2 mmol) in a single portion. The solution was stirred at ambient temperature for 10 min. and quenched by addition of aqueous sodium bicarbonate solution. The layers were separated and the organic phase dried over magnesium sulfate and concentrated under reduced pressure. Flash chromatography on silica gel, eluting with 20% ethyl acetate/hexanes afforded product (9.9 g) as a yellow foam.

### Example 12

### (-)-(4aS)-(10bS)-8-bromo-10b-methyl-1,2,3,4,4a,5,6,10b-octahydrobenzo[f]quinoline-3-one

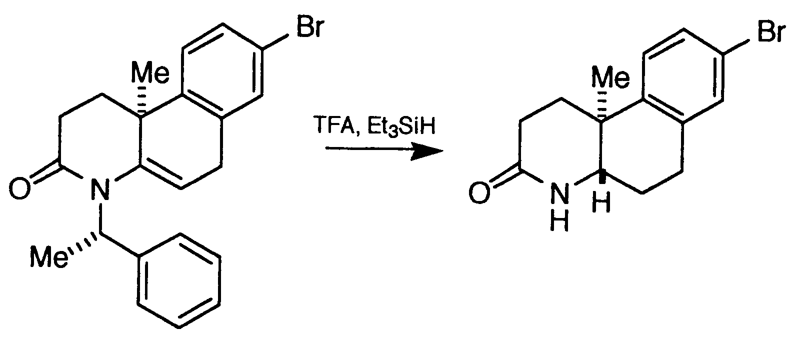

In a 250 mL flask fitted with N₂ inlet, magnetic stirrer and reflux condenser was dissolved enamide (9.9 g, 25 mmol) previously prepared in trifluoroacetic acid (37.5 mL). To the bright yellow solution was added triethylsilane (30 mL) and the mixture heated to reflux for 4.5 H. The solution was cooled to ambient temperature and concentrated under reduced pressure. The crude product was dissolved in ethyl acetate and washed with saturated sodium bicarbonate solution, dried over sodium sulfate and concentrated under reduced pressure. The residue was triturated with pentane (2X) to afford a solid. Recrystallization from ethyl acetate/hexanes afforded product (730 mg) as white crystals. mp = 159°C, m/e 293. a[D]₅₈₉= -99.31° (c=1.0, CHCl₃), a[D]₃₆₅= -338.3° (c=1.0, CHCl₃).

| | Theory | Found |
|---|---|---|
| C | 57.16 | 57.29 |
| H | 5.48 | 5.71 |
| N | 4.76 | 4.74 |

### Example 13

### (+)-(4aR)-(10bR)-8-bromo-10b-methyl-1,2,3,4,4a,5,6,10b-octahydrobenzo[f]quinoline-3-one

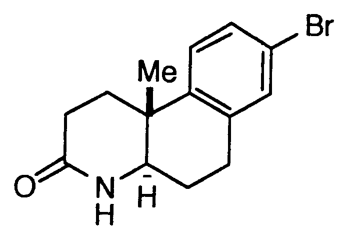

Product was prepared in the manner of Example 11, step 4, an d Example 12, except using R(+)phenethylamine in place of S(-)-phenethylamine. mp = 167°C, m/e 293. a[D]₅₈₉= 112.13° (c=1.0, CHCl₃), a[D]₃₆₅= 304.28° (c=1.0, CHCl₃).

| | Theory | Found |
|---|---|---|
| C | 57.16 | 57.18 |
| H | 5.48 | 5.76 |
| N | 4.76 | 4.50 |

## Claims

1. A heteroannulation process for preparing a non-racemic compound of the formula 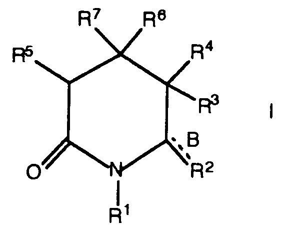 wherein
R¹ represents a chiral auxiliary; line B completes a double bond;
R² represents C₁-C₆(alkylidene); C₁-C₆ aryl(alkylidene); C₃-C₈ cyclo(alkylidene); C₄-C₁₂ cycloalkyl(alkylidene); wherein aryl, alkylidene, and cyclo(alkyl or alkylidene) groups may be substituted with C₁-C₃ alkyl; halo; amino; C₁-C₃ haloalkyl; C₁-C₃ alkyl- or dialkylamino; hydroxy; C₁-C₃ alkoxy; C₁-C₃ alkylthio; cyano; alkoxycarbonyl; oxo; aryloxycarbonyl; alkylsulfonyl; alkylsulfinyl; N- or N,N-dialkylaminocarbonyl; arylaminocarbonyl; alkanoyl or aroyl;
R³ represents C₁-C₆ alkylthio; C₁-C₆ alkoxy; C₁-C₆ alkyl; aryl; C₁-C₆ arylalkyl; C₃-C₈ cycloalkyl; or C₄-C₁₂ cycloalkylalkyl; alkanoyl; aroyl; alkoxycarbonyl; or aryloxycarbonyl; wherein aryl, alkyl and cycloalkyl groups may be substituted with C₁-C₃ alkyl; halo; amino; C₁-C₃ haloalkyl; C₁-C₃ alkyl- or dialkylamino; hydroxy; C₁-C₃ alkoxy; C₁-C₃ alkylthio; cyano; alkoxycarbonyl; oxo; aryloxycarbonyl; alkylsulfonyl; alkylsulfinyl; N- or N,N-dialkylaminocarbonyl; arylaminocarbonyl; alkanoyl or aroyl;
R² and R³ may combine to complete a two-, three- or four-fused-ring structure containing 0-6 heteroatoms chosen from N, O and S; which may be substituted with C₁-C₃ alkyl; halo; amino; C₁-C₃ haloalkyl; C₁-C₃ alkyl- or dialkylamino; hydroxy; C₁-C₃ alkoxy; C₁-C₃ alkylthio; cyano; alkoxycarbonyl; oxo; aryloxycarbonyl; alkylsulfonyl; alkylsulfinyl; N- or N,N-dialkylaminocarbonyl; arylaminocarbonyl; alkanoyl or aroyl;
R⁴ represents C₁-C₆ alkylthio; C₁-C₆ alkoxy; C₁-C₆ alkyl; aryl; C₁-C₆ arylalkyl; C₃-C₈ cycloalkyl; or C₄-C₁₂ cycloalkylalkyl; wherein aryl, alkyl and cycloalkyl groups may be substituted with C₁-C₃ alkyl; halo; amino; C₁-C₃ haloalkyl; C₁-C₃ alkyl- or dialkylamino; hydroxy; C₁-C₃ alkoxy; C₁-C₃ alkylthio; cyano; alkoxycarbonyl; oxo; aryloxycarbonyl; alkylsulfonyl; alkylsulfinyl; N- or N,N-dialkylaminocarbonyl; arylaminocarbonyl; alkanoyl or aroyl;
R³ and R⁴ combine to form a two-, three- or four-ring structure containing 0-6 heteroatoms chosen from N, O and S; which may be substituted with C₁-C₃ alkyl; halo; amino; C₁-C₃ haloalkyl; C₁-C₃ alkyl- or dialkylamino; hydroxy; C₁-C₃ alkoxy; C₁-C₃ alkylthio; cyano; alkoxycarbonyl; oxo; aryloxycarbonyl; alkylsulfonyl; alkylsulfinyl; N- or N,N-dialkylaminocarbonyl; arylaminocarbonyl; alkanoyl or aroyl; provided that when R³ combines with R⁴, R² does not combine with R³;
R⁵ represents hydrogen; C₁-C₆ alkylthio; C₁-C₆ alkoxy; C₁-C₆ alkyl; aryl; C₁-C₆ arylalkyl; C₃-C₈ cycloalkyl; or C₄-C₁₂ cycloalkylalkyl; wherein aryl, alkyl and cycloalkyl groups may be substituted with C₁-C₃ alkyl; halo; amino; C₁-C₃ haloalkyl; C₁-C₃ alkyl- or dialkylamino; hydroxy; C₁-C₃ alkoxy; C₁-C₃ alkylthio; cyano; alkoxycarbonyl; oxo; aryloxycarbonyl; alkylsulfonyl; alkylsulfinyl; N- or N,N-dialkylaminocarbonyl ; arylaminocarbonyl; alkanoyl or aroyl;
R⁶ represents hydrogen; C₁-C₆ alkylthio; C₁-C₆ alkoxy; C₁-C₆ alkyl; aryl; C₁-C₆ arylalkyl; C₃-C₈ cycloalkyl; or C₄-C₁₂ cycloalkylalkyl; wherein aryl, alkyl and cycloalkyl groups may be substituted with C₁-C₃ alkyl; halo; amino; C₁-C₃ haloalkyl; C₁-C₃ alkyl- or dialkylamino; hydroxy; C₁-C₃ alkoxy; C₁-C₃ alkylthio; cyano; alkoxycarbonyl; oxo; aryloxycarbonyl; alkylsulfonyl; alkylsulfinyl; N- or N,N-dialkylaminocarbonyl; arylaminocarbonyl; alkanoyl or aroyl;
R⁷ represents hydrogen; C₁-C₆ alkylthio; C₁-C₆ alkoxy; C₁-C₆ alkyl; aryl; C₁-C₆ arylalkyl; C₃-C₈ cycloalkyl; or C₄-C₁₂ cycloalkylalkyl; wherein aryl, alkyl and cycloalkyl groups may be substituted with C₁-C₃ alkyl; halo; amino; C₁-C₃ haloalkyl; C₁-C₃ alkyl- or dialkylamino; hydroxy; C₁-C₃ alkoxy; C₁-C₃ alkylthio; cyano; alkoxycarbonyl; oxo; aryloxycarbonyl; alkylsulfonyl; alkylsulfinyl; N- or N,N-dialkylaminocarbonyl; arylaminocarbonyl; alkanoyl or aroyl; or
R⁶ and R⁷ combine to form a two-, three- or four-ring structure containing 0-6 heteroatoms chosen from N, O and S; which may be substituted with C₁-C₃ alkyl; halo; amino; C₁-C₃ haloalkyl; C₁-C₃ alkyl- or dialkylamino; hydroxy; C₁-C₃ alkoxy; C₁-C₃ alkylthio; cyano; alkoxycarbonyl; oxo; aryloxycarbonyl; alkylsulfonyl; alkylsulfinyl; N- or N,N-dialkylaminocarbonyl; arylaminocarbonyl; alkanoyl or aroyl;
comprising reacting an enamine of the formula 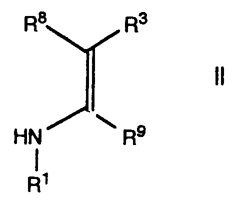 wherein R⁸ represents R⁴; R⁹ represents R², provided that when R² represents an alkylidene-linked group, R⁹ represents the corresponding alkyl-linked group;
with an acryloyl derivative of the formula 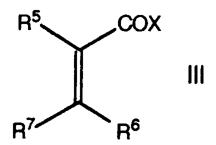 wherein X is a leaving group.

2. A process of Claim 1 for preparing a compound of formula 1 wherein R² and R³ combine to complete a fused-ring structure.

3. A process of Claim 1 for preparing a compound of formula 1 wherein R¹ is 1-phenylethyl.

4. A process of Claim 2 for preparing a compound of formula 1 wherein R¹ is 1-phenylethyl.

5. A process of Claim 1 for preparing a compound of the formula 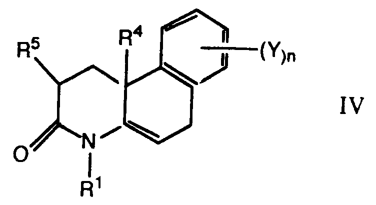 wherein
R¹ is a chiral auxiliary;
R⁵ is hydrogen or C₁-C₄ alkyl;
R⁴ is methyl;
and Y is hydrogen, halo, cyano, trifluoromethyl, C₁-C₃ alkyl, alkoxycarbonyl, aminocarbonyl, alkylaminocarbonyl, dialkylaminocarbonyl, alkylsulfonyl, alkylsulfinyl, C₁-C₃ alkoxy, amino, C₁-C₃ alkyl- or dialkylamino, C₁-C₃ alkylthio or C₁-C₃ haloalkyl;
and n is 1 or 2.
comprising reacting an enamine of the formula 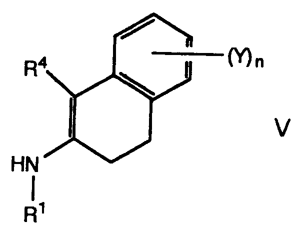 with an acryloyl derivative of the formula 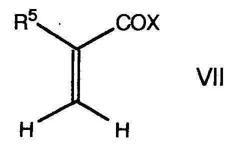 wherein X is a leaving group.

6. A process for cleaving a 1-phenylethyl group from an acylated amino group or from a heterocyclic nitrogen atom adjacent to a carbonyl group comprising reacting the 1-phenylethyl-substituted compound with trifluoroacetic acid.

7. A process of Claim 6 wherein the 1-phenylethyl group is cleared from an acylated amino group.

8. A process of Claim 6 wherein the 1-phenylethyl group is cleaved from a heterocyclic nitrogen atom.

9. A process of Claim 6 wherein the 1-phenylethyl group is cleaved from an unsaturated benzoquinolinone.
